# EUROPÄISCHE PATENTSCHRIFT

(11) **EP 3 911 788 B1**
(45) Veröffentlichungstag und Bekanntmachung des Hinweises auf die Patenterteilung: **11.12.2024**
(21) Anmeldenummer: 20701555.3
(22) Anmeldetag: 17.01.2020
(51) Int. Cl.: D04C 1/06, D04C 3/48

(54) **VERFAHREN ZUM HERSTELLEN EINES GEFLOCHTENEN EINFADENSTENTS, VORRICHTUNG UND FLECHTKERN HIERFÜR**
METHOD FOR MAKING A BRAIDED SINGLE YARN STENT, DEVICE AND MANDREL FOR MAKING SAME
PROCÉDÉ POUR LA RÉALISATION D'UNE TRESSE À FIL UNIQUE, DISPOSITIF ET MANDRIN POUR SON OBTENTION

(30) Priorität: 17.01.2019 DE 102019101238
(43) Veröffentlichungstag der Anmeldung: 24.11.2021
(73) Patentinhaber: STEBO Sondermaschinenbau GmbH & Co. KG, 72411 Bodelshausen (DE)
(72) Erfinder: STEINHILBER, Helmut, 72411 Bodelshausen (DE)
(74) Vertreter: Westphal, Mussgnug & Partner Patentanwälte mbB
(86) Internationale Anmeldenummer: PCT/EP2020/051172
(87) Internationale Veröffentlichungsnummer: WO 2020/148444

(56) Entgegenhaltungen:
- EP-A1- 1 849 440
- WO-A1-2011/137043
- WO-A1-2013/019851
- DE-A1- 102015 214 699

## Beschreibung

Die vorliegende Erfindung betrifft ein Verfahren zum Herstellen eines geflochtenen Einfadenstents mit den Merkmalen des Patentanspruchs 1, einen Flechtkern, zum Durchführen dieses Verfahrens mit den Merkmalen des Patentanspruchs 9 und eine Vorrichtung zum Durchführen des Verfahrens zum Herstellen des geflochtenen Einfadenstents mit den Merkmalen des Patentanspruchs 11.

Aus dem Stand der Technik sind Stents in unterschiedlichen Ausgestaltungen vorbekannt und kommen bevorzugt in Hohlorganen und Gefäßen zum Einsatz. Stents sind tubuläre Endoprothesen, die bevorzugt minimalinvasiv in das Hohlorgan oder das Gefäß implantiert werden können, um ein Lumen in dem Hohlorgan oder dem Gefäß zu stabilisieren und die Funktionsfähigkeit des Hohlorgans aufrecht zu erhalten. Derartige Hohlorgane sind beispielsweise die Atemwege, der Verdauungstrakt sowie Harn- und Gallenwege, sowie das kardiovaskuläre System, wobei sich der Einsatz in diesen oben genannten Organen äußerst bewährt hat.

Aus dem Stand der Technik sind eine Vielzahl von Verfahren zur Herstellung von Stents vorbekannt. Zu diesen Herstellungsverfahren zählen die 2D-Flechttechnik, die 3D-Flechttechnik, die Wickeltechnik und die manuelle Flechttechnik, wobei das jeweils angewendete Herstellungsverfahren einen wesentlichen Einfluss auf die späteren Eigenschaften des erzeugten Stents hat.

Die automatisierbaren Flechttechniken zeichnen sich dadurch aus, dass eine Mehrzahl von Fäden gleichzeitig auf einem Kern abgelegt wird und die Fäden an Kreuzungspunkten insbesondere am Stentende miteinander verbunden werden, um ein Auseinanderfallen des Stents zu verhindern. Aufgrund der vollständigen Automatisierbarkeit der 2D-Flechttechnik und 3D-Flechttechnik haben sich diese schnellen und kostengünstigen Verfahren bei der Herstellung von zylindrischen Stents und Dogbone-Stents bewährt, jedoch haben sich die Stentenden als problematisch erwiesen. An den Stentenden müssen die Fäden durch Schweißverbindungen miteinander verbunden werden, wodurch die Stentenden zu einer traumatischen Belastung des benachbarten Gewebes führen, so dass eine aufwändige Nachbearbeitung zur Vorbeugung notwendig ist. Darüber hinaus hat es sich gezeigt, dass die Stents in dem jeweiligen Hohlorgan einer stetigen dynamischen Belastung und die Schweißverbindungen einer andauernden zyklischen Belastung ausgesetzt sind und die Gefahr besteht, dass bei dem Erreichen von mehreren Zehntausend Zyklen die Schweißverbindungen brechen. Dadurch lässt die stabilisierende Wirkung des Stents nach und weitere Eingriffe in den Patienten können notwendig werden.

Gewickelte Stents sind typischerweise Einfadenstents, welche auf einen Wickelkern in einem zumeist manuellen Prozess gelegt werden, der beispielsweise aus der DE 10 2016 102 503 A1 oder der WO 2017 / 137 577 A1 bekannt ist. Der Wickelkern ist typischerweise ein zylindrischer Kern mit einem proximalen Endbereich und einem dazu beabstandeten distalen Endbereich, wobei in den jeweiligen proximalen Endbereich und in dem jeweiligen distalen Endbereich Umlenkstifte angeordnet sind. Der Einfadenstent wird aus einem Faden mit einem ersten Ende und einem zweiten Ende hergestellt, der bevorzugt aus einer Titan-Nickel-Verbindung, insbesondere Nitinol hergestellt ist. Ausgehend von dem ersten Ende wird der Faden in Windungen spiralförmig von dem proximalen Endbereich zu dem distalen Endbereich und über die Umlenkstifte und zurück zum proximalen Endbereich wiederholt aufgelegt, wobei in Kreuzungspunkten, also an Punkten in denen sich zwei Windungen in einem Winkel schneiden, die jeweilige abgelegte Windung auf die jeweils zuerst abgelegte Windung gelegt wird, wodurch eine sogenannte Überbrückung gebildet wird. Um dem gewickelten Einfadenstent eine ausreichende Festigkeit zu geben und ein Auseinanderfallen des gewickelten Einfadenstents zu verhindern, werden in einer Vielzahl von Kreuzungspunkten die zwei abgelegten Windungen miteinander verschweißt. Auch bei diesen Einfadenstents hat es sich als nachteilig erwiesen, dass die Schweißpunkte sich nachteilig auf die Qualität und Langlebigkeit des Stents auswirken und dass ebenfalls die stabilisierende Wirkung derartiger Stents auf das Hohlorgan nachlässt.

Manuell geflochtene Einfadenstents werden in einem aufwändigen Herstellungsverfahren durch Flechten auf einem Flechtkern hergestellt. Derartige Stents sind Einzelanfertigungen. Zum manuellen Flechten wird ein Flechtkern verwendet, der analog zu dem oben erwähnten Wickelkern ausgebildet ist, jedoch eine Vielzahl von Führungsausnehmungen aufweist, die spiral- oder helixförmig in den Flechtkern eingearbeitet sind. Im Unterschied zu den gewickelten Einfadenstents werden an den Kreuzungspunktes die Windungen abwechselnd in einer Überführung über eine bereits gelegte Windung gelegt und in einer Unterführung unter eine bereits gelegt Windung gesteckt, wodurch ein Geflecht entsteht. Lediglich die beiden Enden des Fadens werden durch eine Schweißverbindung miteinander verbunden. Eine wissenschaftliche Studie "Verfahrensauswahl zur Herstellung metallischer Atemwegstents, Kathrin Kurtenbach, 2016 hat die oben vorgestellten Verfahren verglichen und kommt zu dem Ergebnis, dass manuell geflochtene Einfadenstents den übrigen Stents weit überlegen sind, da insbesondere ein Höchstmaß an Geometrievielfalt und eine besonders flexible Stentstruktur mit atraumatischen Stentenden realisiert werden kann. Manuell geflochtene Einfadenstents weisen keine die Struktur aufrechterhaltende Schweißpunkte auf, wodurch derartige Stents besonders langlebig sind und so zur Patientensicherheit beitragen.

Weiteren Stand der Technik bilden die Druckschriften EP 1 849 440 A1, WO 2011 137 043 A1 und DE 10 2015 214 699 A1.

Als besonders nachteilig an diesem Verfahren hat sich erwiesen, dass die Herstellung manuell geflochtener Einfadenstents hochqualifiziertes Personal erfordert, welches in der Lage sein muss, die Stents in gleichbleibender Qualität herzustellen. Insbesondere das Aufrechterhalten einer konstanten Drahtspannung, das Einfädeln des Fadens in die Führungsausnehmungen in dem Flechtkern erfordert ein Höchstmaß an Konzentration und Erfahrung, wodurch die Herstellung solcher manuell geflochtener Einfadenstents mit erheblichen Kosten verbunden ist. Darüber hinaus hat es sich als nachteilig erwiesen, dass bei dem Einfädeln und Durchziehen des Fadens durch die Führungsausnehmung der Faden teilweise verdrillt wird und die Oberfläche des Fadens durch den Kontakt mit der Führungsausnehmung Oberflächenbeschädigungen erleidet, welche sich nachteilig auf die Langlebigkeit des Stents auswirken.

Hier setzt die vorliegende Erfindung an.

Es ist die Aufgabe der vorliegenden Erfindung, ein Verfahren zum Herstellen eines Einfadenstents zur Verfügung zu stellen, dass in zweckmäßiger Weise ein automatisiertes Herstellen eines geflochtenen Einfadenstents unter Beseitigung der weiteren Nachteile des Standes der Technik ermöglicht. Das Herstellungsverfahren soll insbesondere vollständig oder teilweise automatisierbar sein, um personalintensive Verfahrensschritte durch einen geeigneten Herstellungsprozess zu ersetzen um eine gleichbleibende und höherwertige Qualität mit geringerem bzw. vernachlässigbarerem Ausschuss herzustellen. Darüber hinaus soll ein Flechtkern sowie eine Vorrichtung zur Durchführung des Verfahrens vorgeschlagen werden, die ein voll automatisiertes Verfahren ermöglichen sollen.

Diese Aufgaben werden erfindungsgemäß durch ein Verfahren zur Herstellung eines geflochtenen Einfadenstents mit den Merkmalen des Patentanspruchs 1, durch einen Flechtkern mit den Merkmalen des Patentanspruchs 9 und durch eine Vorrichtung zur Durchführung des erfindungsgemäßen Verfahrens mit den Merkmalen des Patentanspruchs 11 gelöst.

Für die Durchführung des erfindungsgemäßen Verfahrens mit den Merkmalen des Patentanspruchs 1 wird ein Faden mit einem ersten Ende und einem zweiten Ende sowie ein Flechtkern bereitgestellt, wobei der Flechtkern einen proximalen Endbereich und einen in der Längsachse zu dem proximalen Endbereich beabstandet angeordnete distalen Endbereich aufweist. In dem proximalen Endbereich ist wenigstens eine erste Umlenkung und in dem distalen Endbereich wenigstens eine zweite Umlenkung angeordnet. Zwischen dem proximalen Endbereich und dem distalen Endbereich weist der Flechtkern eine Vielzahl von Führungsausnehmungen auf, welche im Wesentlichen senkrecht zu der Längsachse eingearbeitet oder eingeformt ist. Der Faden wird ausgehend von dem ersten Ende auf den Flechtkern spiral- oder helixförmig in Windungen zwischen der wenigstens einen ersten Umlenkung und der wenigstens einen zweiten Umlenkung abgelegt, wobei der Faden an mindestens einem Kreuzungspunkt über eine bereits gelegte Windung in einer Überführung gelegt wird und der der Faden an mindestens einem Kreuzungspunkt in eine Unterführung unter einer bereits gelegten Windung gesteckt ist, wobei das erfindungsgemäße Verfahren folgende Verfahrensschritte zur Bildung einer Unterführung beinhaltet
- Legen einer Windung auf den Flechtkern bis zum Erreichen eines Kreuzungspunktes,
- Einführen und Durchstecken des zweiten Endes des Fadens in einer Steckrichtung durch die Führungsausnehmung in dem Flechtkern, und
- Entgegennehmen und Durchziehen des Fadens durch die Führungsausnehmung.

Erfindungsgemäß ist die Erkenntnis wesentlich, dass beim Stecken des Fadens zum Bilden einer Unterführung das freie Ende des Fadens nicht entlang der spiral- oder helixförmigen Windung geführt ist, sondern im Wesentlichen senkrecht zu der Längsachse des Flechtkerns, wodurch das Einfädeln, das Entgegennehmen und das Durchziehen des Fadens erheblich erleichtert ist. Die Führungsausnehmung, die im Wesentlichen senkrecht zu der Längsachse angeordnet ist, erlaubt es, dass das freie Ende des Fadens entlang einer Steckrichtung entlang einer Geraden, welche sekantial zu dem Flechtkern angeordnet ist, durch die Führungsausnehmung gesteckt wird, währenddessen die aus dem Stand der Technik bekannten Flechtkerne es erfordern, dass das freie Ende des Fadens entlang einer gekrümmten (helixförmigen) Kurve durch die Führungsausnehmung gesteckt wird. Im Zusammenhang mit dieser Erfindung kann unter einer Ausrichtung, die im Wesentlichen senkrecht ausgerichtet ist, eine Winkeltoleranz bevorzugt ±2,5° und ganz besonders bevorzugt ±1° verstanden werden. Unter sekantial kann im Kontext dieser Erfindung eine ordnung einer als Sekante ausgebildeten Geraden verstanden werden, die in zwei Punkten die Mantelfläche des Flechtkerns schneidet und beabstandet zu dem Mittelpunkt bzw. der Längsachse angeordnet ist.

Gemäß einer weiteren vorteilhaften Ausführung des vorliegenden erfindungsgemäßen Verfahrens ist es vorgesehen, dass der Faden durch die Führungsausnehmung so lange durchgezogen wird, bis die Windung des Fadens mit einer vorgegebenen und bevorzugt konstanten Spannung auf dem Flechtkern aufgelegt ist. Durch das Ziehen an dem Faden wird bevorzugt der Faden derart auf dem Flechtkern positioniert, dass dieser in seiner endgültigen Position der Windung an diesem anliegt. Durch das Anziehen liegt der Faden nicht auf dem Grund der Führungsausnehmung, sondern überbrückt bzw. überspannt zugspannungsbedingt die Führungsausnehmung, wodurch die endgültige Positionierung auf dem Flechtkern realisiert werden kann.

Eine weitere vorteilhafte Ausgestaltung der vorliegenden Erfindung sieht vor, dass die Steckrichtung parallel zu der Führungsausnehmung ausgerichtet ist. Weiter bevorzugt ist es, wenn das freie Ende des Fadens in Steckrichtung zentrisch zu der Führungsausnehmung eingeführt und durchgesteckt wird. Dadurch kann eine möglichst kontaktfreie Führung des Fadens in der Führungsausnehmung realisiert werden, ohne dass es zu Beschädigungen an dem Faden an der Führungsausnehmung des Flechtkerns durch Reibkontakt kommen kann.

Nach Maßgabe einer weiteren vorteilhaften Ausführung des erfindungsgemäßen Verfahrens hält ein erster Greifer den Faden in einem Abstand zu dem freien Ende und steckt das freie Ende zur Bildung einer Unterführung in Steckrichtung in die Führungsausnehmung ein. Der Abstand zwischen dem freien Ende und dem ersten Greifer entspricht mindestens der Länge der Führungsausnehmung (in Steckrichtung) bzw. mindestens ein Drittel des Durchmessers des Flechtkerns. Bevorzugt ist der Abstand größer als der Durchmesser des Flechtkerns. Der erste Greifer kann CNC gesteuert und relativ zu dem Flechtkern beweglich sein, wodurch eine exakte Positionierung des freien Endes relativ zu der jeweiligen Führungsausnehmung erfolgen kann. Der erste Greifer hält währenddessen den Faden derart, dass dieser in seiner Lage fixiert ist und unter seiner Kontrolle keine Veränderung der Lage des Fadens erfolgen kann, z. B eine ungewünschte Verschiebung oder ein Verdrillen. Nach dem Durchstecken des Fadens kann der erste Greifer den Faden loslassen und auf der in Steckrichtung hinter der Führungsausnehmung angeordneten Seite den Faden wieder aufnehmen bzw. entgegennehmen.

Weiterhin ist es besonders vorteilhaft, wenn der erste Greifer oder ein zweiter Greifer vorgesehen ist, der eingerichtet ist, das freie Ende des Fadens in Steckrichtung hinter der Führungsausnehmung entgegen zu nehmen. Der zweite Greifer kann den ersten Greifer beinhalten.

Darüber hinaus kann der zweite Greifer analog zu dem erste Greifer ausgebildet sein und CNC gesteuert sein, wodurch eine exakte Positionierung relativ zu dem Flechtkern zur Entgegennahme des durch die Führungsausnehmung durchgesteckten freien Endes des Fadens auf der dem ersten Greifer gegenüberliegenden Seite der Führungsausnehmung erfolgen kann.

Besonders ist es bei der Durchführung des erfindungsgemäßen Verfahrens vorteilhaft, wenn der erste Greifer den Faden mindestens solange hält, bis der zweite Greifer das freie Ende des Fadens ebenfalls hält. Sobald der zweite Greifer das freie Ende des Fadens übernommen hat und hält, kann der erste Greifer den Faden loslassen. Dadurch ist sichergestellt, dass die Kontrolle über den Faden bei der Durchführung des erfindungsgemäßen Verfahrens ununterbrochen beibehalten wird und beispielsweise ein ungewünschtes Verdrillen des Fadens nicht möglich ist.

Eine weitere vorteilhafte Weiterbildung des erfindungsgemäßen Verfahrens sieht vor, dass das Durchziehen des Fadens ausgehend von dem freien Ende durch ein Aufwickeln auf eine Spule um eine zweite Achse erfolgt. Es ist bevorzugt, wenn wenigstens einer der Greifer, vorzugsweise der zweite Greifer, der das freie Ende hinter der Führungsausnehmung entgegennimmt, zusammen mit der Spule um die zweite Achse zum Durchziehen und Aufwickeln des Fadens durch die Führungsausnehmung rotiert, wodurch auch beim Aufwickeln des Fadens vollständig die Kontrolle über die Lage des Fadens bewahrt werden kann. Weiterhin ist es besonders bevorzugt, wenn zunächst der zweite Greifer das freie Ende des Fadens in der Steckrichtung durch die Führungsausnehmung zieht und der erste Greifer der Zustellbewegung des zweiten Greifer folgt und den Faden ebenfalls in Steckrichtung hinter der Führungsausnehmung wieder aufnimmt und hält. Anschließend kann der erste Greifer zusammen mit dem zweiten Greifer und der Spule um die zweite Achse zum vollständigen Durchziehen des Fadens durch die Führungsausnehmung verdreht werden und den Faden aufwickeln.

Besonders bevorzugt ist es, wenn beim Aufwickeln des Fadens auf die Spule oder beim Legen des Fadens auf den Flechtkern die vorgegebene Spannung des Fadens auf dem Flechtkern überwacht ist, beispielsweise durch mindestens einen Kraft- und/oder Drehmomentsensor, durch den das an der Spule anliegende Drehmoment bzw. die Spannung bzw. die Zugkraft an dem Faden erfasst wird.

Eine besonders vorteilhafte Ausgestaltung der vorliegenden Erfindung sieht vor, dass die zweite Achse entweder parallel zu der Längsachse des Flechtkerns ausgerichtet ist, oder dass die zweite Achse senkrecht zu der Längsachse des Flechtkerns angeordnet ist.

Auch kann es bei der Durchführung des erfindungsgemäßen Verfahrens vorteilhaft sein, wenn der Faden nach dem Durchziehen durch die Führungsausnehmung zur Bildung einer Unterführung auf dem Flechtkern gehalten ist. Besonders bevorzugt ist es, wenn mindestens ein Haltemittel vorgesehen ist, dass eingerichtet ist, eine Haltekraft auf die Windung des Fadens aufzubringen. Das mindestens eine Haltemittel kann wenigstens eine auf dem Flechtkern abgelegte Windung fixieren und das Legen der Windung bis zum Erreichen der nächsten Unterführung fixieren.

Es hat sich weiterhin als vorteilhaft erwiesen, wenn der Faden vor dem Ein- und Durchstecken des freien Endes des Fadens zur Bildung der Unterführung vollständig von der Spule abgewickelt wird. Nach dem Abwickeln der Spule wird das freie Ende des Fadens in Steckrichtung vor der nächsten Führungsausnehmung zur Bildung einer weiteren Unterführung positioniert. Hierzu kann bevorzugt der Flechtkern in der Längsachse verdreht werden und eine entsprechende Lateralbewegung entlang der Längsachse zwischen dem proximalen Endbereich und dem distalen Endbereich des Flechtkerns vorgenommen werden, um entsprechend der Steigung der Windung das freie Ende des Fadens vor der entsprechenden Führungsausnehmung zu positionieren.

Darüber hinaus ist es vorteilhaft, wenn an dem zweiten Ende des Fadens eine Führungseinrichtung angeordnet ist, die eine größere Steifigkeit aufweist als der Faden. Die Führungseinrichtung kann beispielsweise nach Art des Nadel- und Fadenprinzips den Faden führen, wobei der erste Greifer und der zweite Greifer nicht den Faden sondern die Führungseinrichtung halten können, wodurch die Handhabung des Fadens beim Durchführen des erfindungsgemäßen Verfahrens erheblich vereinfacht ist. Besonders ist es bevorzugt, wenn die Form der Führungseinrichtung an die Form der Führungsausnehmung angepasst ist. Die Führungseinrichtung und die Führungsausnehmung können beispielsweise einen mehreckigen Querschnitt aufweisen. Dadurch kann die Führungseinrichtung in der Führungsausnehmung durch einen Formschluss (mit einer Spielpassung) nicht in der Führungsausnehmung verdreht werden, wodurch ein ungewolltes Verdrillen des Fadens beim Erzeugen einer Unterführung verhindert ist. Die Führungseinrichtung kann bei dem Bereitstellen des Fadens an dem Faden befestigt werden.

Eine weitere vorteilhafte Ausgestaltung der vorliegenden Erfindung sieht vor, dass das erste Ende und das zweite Ende des Fadens in einer gemeinsamen Windung miteinander verbunden werden. Besonders bevorzugt ist die Verschweißung zwischen dem proximalen Endbereich und dem distalen Endbereich mittig angeordnet. Noch bevorzugter ist es, wenn die Verschweißung in einer Unterführung angeordnet ist. Die Verschweißung kann bevorzugt mittels Elektronenstrahlschweißen, Laserschweißen, Widerstandsschweißen, Reibschweißen oder Mikroplasmaschweißen erzeugt werden. Nach dem Verschweißen können die freien Endbereiche des ersten Endes und des zweiten Endes des Fadens jenseits der Verschweißung abgetrennt werden. Somit befindet sich einerseits die Verschweißung in einem Bereich mit geringen mechanischen Belastungen und andererseits ist die Verschweißung atraumatisch positioniert.

Es hat sich als besonders vorteilhaft erwiesen, dass der gelegte Faden aufgrund der Fadenspannung aus der jeweiligen Führungsausnehmung herausgezogen wird und die Führungsausnehmung überbrückt bzw. überspannt. Dadurch wird die Führungsausnehmung freigegeben und der Faden kann mehrfach entlang der Windungen nach dem oben beschriebenen Verfahren durch die wenigstens eine Führungsausnehmung zur Bildung einer Unterführung durchgesteckt werden. In dieser Windung liegen dann mindestens zwei Abschnitte des Fadens unmittelbar nebeneinander. Insbesondere ist das Verbinden des ersten Endes und des zweiten Endes des Fadens in einer gemeinsamen Windung mit zwei Abschnitten erheblich vereinfacht. Auch können die Abschnitte in einer oder mehreren Windungen miteinander verdrillt werden. Weiterhin ist es vorteilhaft, wenn der geflochtene Einfadenstent nach dem Flechten durch Tempern und/oder Elektropolieren nachbehandelt wird. Bevorzugt wird der Verfahrensschritt Tempern durchgeführt, bevor der Einfadenstent von dem Flechtkern genommen wird. Weiterhin ist es vorteilhaft, wenn der Einfadenstent nach dem Abnehmen von dem Flechtkern elektropoliert wird, wodurch auch geringste Oberflächenbeschädigungen, insbesondere Kratzer entfernt werden. Dadurch wird die Dauerfestigkeit des geflochtenen Einfadenstents erhöht.

Es hat sich des Weiteren als vorteilhaft erwiesen, wenn zur Herstellung des geflochtenen Einfadenstents ein metallischer oder ein aus einem Polymer hergestellter Faden verwendet wird. Der Faden kann besonders bevorzugt ein Einzelfaden sein. Alternativ kann der Faden ein aus mehreren Einzelfäden (Litzen) hergestellter Faden sein, wobei die Litzen aus unterschiedlichen Werkstoffen hergestellt sein können. Bevorzugt werden für den Faden folgende Werkstoffe oder Werkstoffgruppen verwendet: Nickel-Titan-Legierungen, insbesondere Nitinol, Magnesium-Legierungen, Edelstahl, insbesondere medizinischer Edelstahl (z.B. 316LVM ), Chrom-Legierungen, Kobalt-Chrom-Legierungen, Kobalt-Legierungen, Niobium enthaltende Legierungen, Platin-Legierungen, Platin-Chrom-Legierung, Tantal-Legierungen. Auch kann zur Herstellung des geflochtenen Einfadenstents ein aus einem Polymer oder Polymergemisch hergestellter Faden verwendet werden.

Es ist vorteilhaft, wenn zur Herstellung des Einfadenstents ein Faden mit einem Durchmesser im Bereich von 1 µm bis 1000 µm verwendet wird, vorzugsweise im Fall eines Blutgefäß-Einfadenstents im Bereich zwischen 10 µm und 350 µm und vorzugsweise im Fall eines pulmonalen, trachealen, laryngealen, ureteralen, Magen-Darm- oder Gallengang-Einfadenstents im Bereich zwischen 10 µm und 500 pm.

Bevorzugt werden die spiralförmigen Windungen derart auf den Wickelkern gelegt, dass ein Kreuzungswinkel α, in dem sich zwei Windungen in einem Kreuzungspunkt schneiden bzw. kreuzen, im Bereich zwischen 10°≤ α ≤ 80°, vorzugsweise im Bereich zwischen 30° ≤ α ≤ 60° liegt.

Ein zweiter und weiterer Aspekt der vorliegenden Beschreibung betrifft einen Einfadenstent, hergestellt durch ein erfindungsgemäßes Verfahren.

Ein dritter Aspekt der vorliegenden Erfindung betrifft einen Flechtkern, zur Durchführung des erfindungsgemäßen Verfahrens, aufweisend einen Kern mit einer Längsachse, einem proximalen Endbereich und einem distalen Endbereich, wobei der proximale Endbereich in der Längsachse beabstandet zu dem distalen Endbereich angeordnet ist. Weiterhin umfasst der Flechtkern wenigstens eine erste Umlenkung und wenigstens eine zweite Umlenkung, wobei die wenigstens eine erste Umlenkung in dem proximalen Endbereich angeordnet ist und die wenigstens eine zweite Umlenkung in dem distalen Endbereich angeordnet ist. Zwischen dem proximalen Endbereich und dem distalen Endbereich ist erfindungsgemäß eine Vielzahl von Führungsausnehmungen angeordnet, wobei die jeweilige Führungsausnehmung im Wesentlichen senkrecht zu der Längsachse angeordnet ist. Im Zusammenhang mit dieser Erfindung wird unter einer Anordnung, welche im Wesentlichen senkrecht zu der Längsachse ausgerichtet ist, eine Winkeltoleranz von bevorzugt weniger als ±2,5° und am meisten bevorzugt weniger als ±1° zwischen der Ausrichtung der wenigstens einen Führungsausnehmung und der Längsachse verstanden. Die Führungsausnehmung ist in den Kern eingearbeitet oder eingeformt und nach Art einer Sekante und als senkrecht zu der Längsachse ausgerichtete Nut ausgebildet. Die Tiefe und Breite der Führungsausnehmung ist an die Größe des Fadens angepasst und mindestens genauso groß, wie der durch die Führungsausnehmung durchzuführende Faden. Die Breite der Führungsausnehmung ist durch eine Maschenweite begrenzt, wobei die Maschenweite den Abstand zwischen zwei Kreuzungspunkten parallel zu der Längsachse beschreibt.

Gemäß einer weiteren vorteilhaften Ausgestaltung der vorliegenden Erfindung ist der Kern zylinderförmig ausgebildet. Insbesondere ist es bevorzugt, wenn der Kern zwischen dem proximalen Endbereich und dem distalen Endbereich einen konstanten Durchmesser aufweist oder wenn der Kern zwischen dem proximalen Endbereich und dem distalen Endbereich zunächst einen sich verringernden und anschließend wieder einen sich vergrößernden Durchmesser aufweist, wodurch ein geflochtener Einfadenstent in Dogbone-Shape hergestellt werden kann.

Nach Maßgabe einer weiteren vorteilhaften Ausgestaltung der vorliegenden Erfindung ist die wenigstens eine Umlenkung durch einen von dem Kern, bevorzugt radial, abstehenden Umlenkstift ausgebildet. Die Umlenkstifte können an dem Kern des Flechtkerns lösbar befestigt sein, wodurch zum Abnehmen des geflochtenen Einfadenstents die Umlenkstifte in dem proximalen Endbereich und in dem distalen Endbereich abgenommen werden können und der Einfadenstent von dem Flechtkern abgezogen bzw. abgenommen werden kann. Die Umlenkstifte können als Klammer, Bogen, T-förmiger Pin oder dergleichen ausgeführt sein.

Darüber hinaus hat es sich als vorteilhaft erwiesen, wenn in dem proximalen Endbereich und in dem distalen Endbereich jeweils eine Anzahl i von Umlenkungen angeordnet ist. Besonders bevorzugt ist es, wenn Umlenkungen in dem proximalen Endbereich und in dem distalen Endbereich umfangssymmetrisch angeordnet sind. Die Anzahl i von Umlenkungen in dem proximalen Endbereich und in dem distalen Endbereich kann bevorzugt eine gerade Zahl sein. Eine besonders bevorzugte Ausgestaltung des Flechtkerns sieht vor, dass in dem proximalen Endbereich und in dem distalen Endbereich jeweils zehn oder zwölf Umlenkungen (i =10 oder i=12) umfangssymmetrisch angeordnet sind.

Es ist vorteilhaft, wenn die wenigstens eine Führungsausnehmung eine in den Kern sekantial eingearbeitete oder eingeformte Ausnehmung ist.

Bevorzugt ist es, wenn über den Umfang des Kerns in einer Ebene quer zu der Längsachse eine Anzahl j von Führungsausnehmungen ausgebildet ist. Die Führungsausnehmungen können beabstandet zueinander ausgebildet sein, wodurch zwischen den Führungsausnehmungen die aufgelegten Windungen nicht beeinflusst werden. Weiterhin ist es vorteilhaft, wenn entlang der Längsachse die Führungsausnehmungen in einer Anzahl k reihenweise angeordnet sind.

Vorteilhaft ist es, wenn die wenigstens eine Führungsausnehmung im Querschnitt einen rechteckigen, einen U- oder V-förmigen Querschnitt, oder Kombinationen dieser aufweist. Insbesondere hat es sich als vorteilhaft erwiesen, wenn die Führungsausnehmung eine veränderliche Breite aufweist. Besonders bevorzugt nimmt die Breite der Führungsausnehmung ausgehend von einem Mittelpunkt der Führungsausnehmung weiter bevorzugt in beide Richtungen zu, wodurch das Einfädeln und das Einstecken des freien Endes des Fadens vereinfacht ist und der Positionierungsaufwand bei dem Ein- und Durchstecken des freien Endes des Fadens weiter reduziert werden kann.

Ein vierter Aspekt der vorliegenden Erfindung betrifft eine Vorrichtung zum Durchführen des erfindungsgemäßen Verfahrens zum Herstellen eines geflochtenen Einfadenstents. Die Vorrichtung zum Durchführen des Verfahrens umfasst eine erste Achse und eine zweite Achse, wobei die erste Achse eingerichtet ist, einen erfindungsgemäßen Flechtkern zu halten bzw. aufzunehmen. Weiterhin umfasst die Vorrichtung wenigstens einen Arm mit einem ersten Greifer, wobei der wenigstens eine Greifer eingerichtet ist, einen Faden zu greifen und zu halten. Der wenigstens eine Arm und wenigstens eine Spule sind in der zweiten Achse drehbar angeordnet. Erfindungsgemäß ist vorgesehen, dass der Flechtkern um die ersten Achse drehbar gehalten ist, wobei die Vorrichtung bevorzugt die Drehung des Flechtkerns um die erste Achse steuert. Weiterhin steuert die Vorrichtung die zweite Achse derart an, dass die Spule und der Arm gemeinsam in der zweiten Achse gedreht werden. Bei einer Rotation um die zweite Achse kann der Arm bzw. dessen wenigstens einer Greifer das freie Ende des Fadens halten, während gleichzeitig der Faden auf die Spule aufgewickelt oder von der Spule abgewickelt wird.

Gemäß einer weiteren vorteilhaften Ausgestaltung der vorliegenden Erfindung umfasst der wenigstens eine Arm den ersten Greifer und einen zweiten Greifer, die zueinander beabstandet angeordnet sind und gemeinsam um die zweite Achse drehbar sind. Der erste Greifer und der zweite Greifer können jeweils getrennt durch eine Steuerung der Vorrichtung angesteuert werden, so dass der erste Greifer und/oder der zweite Greifer jeweils unabhängig eine Bewegung zum Aufnehmen, Greifen und Halten des Fadens durchführen können bzw. kann.

Nach Maßgabe einer vorteilhaften Ausgestaltung der erfindungsgemäßen Vorrichtung zum Herstellen eines geflochtenen Einfadenstents weisen bzw. weist die erste Achse und/oder die zweite Achse mindestens einen Kraftsensor auf, der eingerichtet ist, das an der ersten Achse und/oder der zweiten Achse anliegende Drehmoment bzw. eine Querkraft zu bestimmen. Durch das Bestimmen des an der ersten Achse und/oder der zweiten Achse anliegenden Drehmoments bzw. der an der ersten Achse und/oder der zweiten Achse anliegenden Querkraft kann die Zugspannung bestimmt werden, mit der an dem Faden bei dem Legen auf den Flechtkern gezogen wird, wodurch sichergestellt werden kann, dass der Faden beim Auflegen auf den Flechtkern mit einer konstanten und vorbestimmten Zugspannung aufgelegt ist.

Gemäß einer weiteren Ausgestaltung der vorliegenden Erfindung kann es vorteilhaft sein, dass der wenigstens eine Arm relativ zu der ersten Achse beweglich ist. Der wenigstens eine Arm ist bevorzugt durch die Steuerung der Vorrichtung CNC gesteuert, wodurch der jeweilige wenigstens eine Greifer exakt positioniert werden kann. Dies ist zum Beispiel erforderlich um einerseits den Greifer in einer Steckrichtung vor dem Flechtkern und der Führungsausnehmung zu positionieren und andererseits in Steckrichtung hinter dem Flechtkern um das freie Ende des Fadens entgegenzunehmen. Während der erste Greifer bevorzugt eingerichtet ist, ein von dem Greifer abstehendes freies Ende des Fadens zur Bildung einer Unterführung in einem Kreuzungspunkt zu führen, ist der zweite Greifer bevorzugt eingerichtet, den Faden bzw. dessen freies Ende nach der Bildung der Unterführung entgegenzunehmen und durch die Unterführung durchzuziehen. Der erste Greifer und der zweite Greifer können in einer besonders bevorzugten Ausgestaltung der vorliegenden Erfindung nach Bildung der Unterführung den Faden gemeinsam halten und zusammen mit der Spule um die zweite Achse gedreht werden, wodurch der Faden beim Aufwickeln um die Spule vor einer Änderung der Lage gesichert ist und es verhindert werden kann, dass der Faden verdrillt ist.

Bevorzugt ist es, wenn die erste Achse und die zweite Achse parallel zueinander ausgerichtet sind oder wenn die erste Achse und die zweite Achse senkrecht zueinander angeordnet sind.

Darüber hinaus sieht eine Maßgabe der vorliegenden Erfindung vor, dass die Vorrichtung wenigstens ein Haltemittel umfasst, das eingerichtet ist, eine Haltekraft auf den Flechtkern aufzubringen. Insbesondere ist es bevorzugt, wenn das Haltemittel eine auf den Flechtkern aufgelegten Windung auf dem Flechtkern fixieren kann, so dass die Spannung des Fadens der bereits auf den Flechtkern aufgelegten Windungen nicht verloren geht. Besonders bevorzugt ist es, wenn das mindestens eine Haltemittel um die erste Achse drehbar ist, wobei weiterhin besonders bevorzugt ist, wenn das wenigstens eine Haltemittel zusammen mit der ersten Achse bzw. mit dem in der ersten Achse angeordneten Flechtkern um die erste Achse gedreht werden kann.

Nachfolgend wird unter Bezugnahme auf die begleitenden Zeichnungen ein erfindungsgemäßes Ausführungsbeispiel der vorliegenden Erfindung im Detail erläutert. Es zeigen:
- Figur 1: eine stark vereinfachte Darstellung einer erfindungsgemäßen Vorrichtung zum Herstellen eines geflochtenen Einfadenstents mit einem Flechtkern, wobei der Flechtkern eine Vielzahl von Führungsausnehmungen aufweist, die zur Bildung einer Unterführung in einem Kreuzungspunkt senkrecht in dem Flechtkern ausgebildet sind,
- Figuren 2a bis 2g: schematische und vereinfachte Detaildarstellungen einzelner Verfahrensschritte zur Bildung der Unterführung mit der Vorrichtung gemäß Figur 1, und
- Figur 3: eine vergrößerte Draufsicht auf eine Führungsausnehmung gemäß Figur 1 oder 2, und
- Figuren 4a bis 4c: schematische Schnittdarstellungen durch die Führungsausnehmung.

Gleiche Bezugsziffern bezeichnen auch in den unterschiedlichen Darstellungen gleiche oder gleichartige Teile. Zur besseren Übersicht sind nicht sämtliche Bezugsziffern in sämtlichen Figuren angegeben.

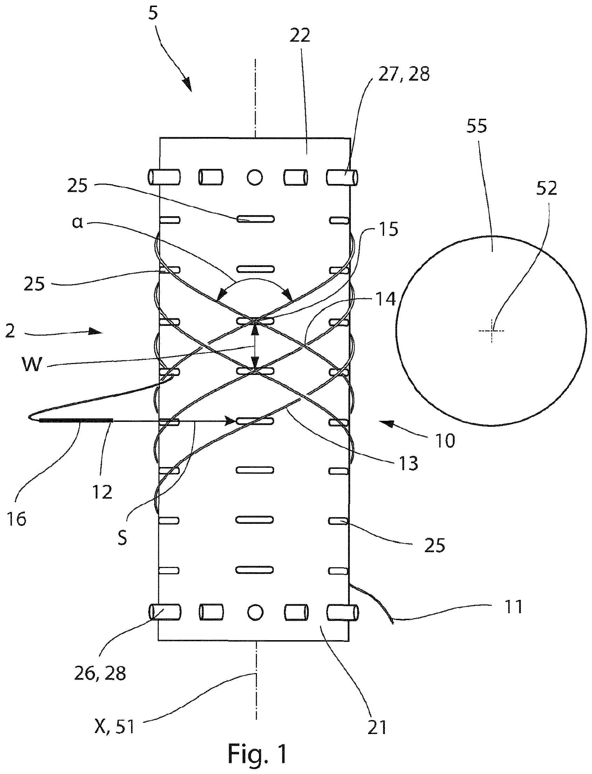

Figur 1 zeigt die Vorrichtung 5 zum Herstellen eines geflochtenen Einfadenstents aus einem Faden 10. Die Vorrichtung 5 umfasst eine erste Achse 51 und eine zweite Achse 52, die in dem dargestellten und exemplarischen Ausführungsbeispiel senkrecht zueinander ausgerichtet sind, aber ebenso parallel zueinander ausgerichtet sein können. Die erste Achse 51 und die zweite Achse 52 können Maschinenachsen entsprechen und können jeweils von einer Steuerung der Vorrichtung 5 voneinander unabhängig angesteuert werden.

Zur Herstellung des geflochtenen Einfadenstents wird ein Flechtkern 2 mit der Vorrichtung 5 in der ersten Achse 51 gekoppelt, wodurch der Flechtkern 2 in der Vorrichtung 1 mit der entsprechenden Maschinenachse der Vorrichtung trieblich gekoppelt ist und um die erste Achse 51 drehbar gehalten ist.

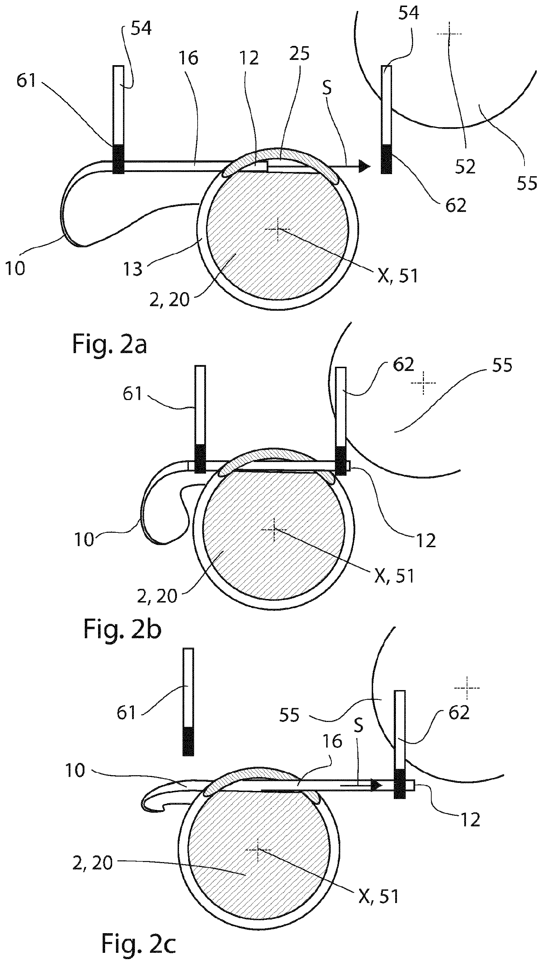

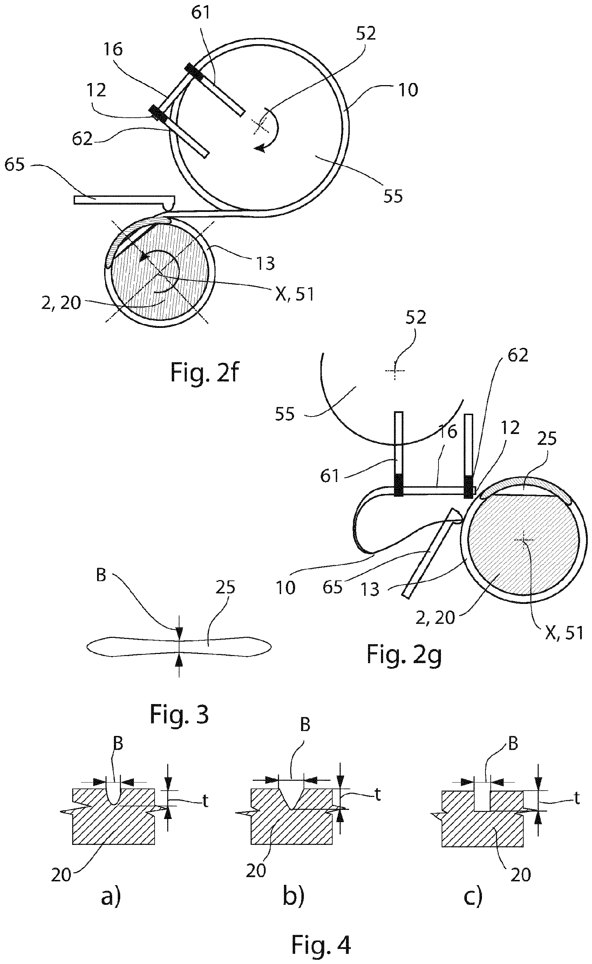

In der zweiten Achse 52 sind ein Arm 54, siehe Figuren 2a bis 2g, und eine Spule 55 drehbar gehalten, wobei die zweite Achse 52 relativ zu der ersten Achse 51 beweglich ist. Der Arm 54 umfasst einen ersten Greifer 61 und einen zweiten Greifer 62, die voneinander unabhängig von der Steuerung der Vorrichtung gesteuert werden können und eingerichtet sind, den Faden 10 zu greifen und zu halten. Durch den Arm 54 können die Greifer 61, 62 relativ zueinander und mit dem Arm 54 gemeinsam relativ zu der ersten Achse 51 positioniert werden.

Der Flechtkern 2 umfasst einen zylindrischen Kern 20 mit einer Längsachse X, einem proximalen Endbereich 21 und einem distalen Endbereich 22. Der proximale Endbereich 21 und die distale Seite 22 sind parallel und beabstandet in der Längsachse X angeordnet. Der proximale Endbereich 21 weist eine Mehrzahl von ersten Umlenkungen 26 auf und der distale Endbereich 22 eine Mehrzahl von zweiten Umlenkungen 27, wobei die jeweilige Umlenkung 26, 27 als ein von einer Mantelfläche des zylindrischen Kerns 20 abstehender Umlenkstift 28 ausgebildet ist. Der jeweilige Umlenkstift 28 kann eine Klammer, ein Bogen, ein Bügel oder ein T-förmiger Pin sein.

In dem dargestellten Ausführungsbeispiel sind über den Umfang auf der Mantelfläche sowohl in dem proximalen Endbereich 21 als auch in dem distalen Endbereich 22 jeweils eine Anzahl i von zwölf Umlenkstiften 28 umfangssymmetrisch angeordnet, wobei die Umlenkstifte 28 lösbar an dem zylindrischen Kern 20 befestigt sind. Die Anzahl i kann beliebig gewählt sein und die Anzahl i der ersten Umlenkungen 26 und der zweiten Umlenkungen 27 sollen identisch sein, wobei bevorzugt die Anzahl i eine gerade Zahl ist. Die Umlenkstifte 28 sind bevorzugt zylindrisch ausgebildet und stehen bevorzugt radial von der Mantelfläche des Kerns 20 ab.

Weiterhin ist in Figur 1 dargestellt, dass zwischen dem proximalen Endbereich 21 und dem distalen Endbereich 22 zwischen der ersten Umlenkung 26 und der zweiten Umlenkung 27 eine Vielzahl von Führungsausnehmungen 25 spaltenweise angeordnet ist, die im Wesentlichen senkrecht zu der Längsachse X ausgerichtet sind. Die jeweilige Führungsausnehmung 25 ist entlang einer Sekante in den Kern 20 ausgebildet, wobei die Sekante definitionsgemäß die Mantelfläche des bevorzugt zylindrischen Kerns 20 an zwei Punkten schneidet und entsprechend zwischen einer Tangente und der Längsachse X angeordnet ist. Umfangssymmetrisch um die Längsachse X sind jeweils in einer Ebene senkrecht zu der Längsachse eine Anzahl j von Führungsausnehmungen 25 angeordnet, wobei die Anzahl j der Hälfte der Anzahl i von Umlenkungen 26, 27 entspricht, also j = i/2.

Die Führungsausnehmungen 25 sind in der Längsachse X spaltenweise in einer Anzahl k angeordnet, wobei die Anzahl k von einer Maschenweite W und einem Abstand zwischen dem proximalen Endbereich 21 und dem distalen Endbereich 22 abhängt, welcher einer Länge des zu flechtenden Einfadenstents entspricht.

Die jeweilige Führungsausnehmung 25 kann als tangential bzw. sekantial verlaufende Nut oder Ausnehmung senkrecht zur der Längsachse X in die Mantelfläche des Kerns 20 eingearbeitet oder eingeformt sein

Die jeweilige Führungsausnehmung 25 weist eine Breite B und eine Tiefe T auf, und kann im Querschnitt eine beliebige Form aufweisen, wobei drei typische Querschnitte für die Führungsausnehmung 25 in den Figuren 4a bis 4c dargestellt sind. Die Führungsausnehmung 25 kann beispielsweise im Querschnitt einen U-förmigen Querschnitt aufweisen, dargestellt in Figur 4a. Auch kann die Führungsausnehmung 25 einen V-förmigen Querschnitt - siehe Figur 4b - oder einen rechteckigen Querschnitt - siehe Figur 4c - aufweisen. Die Breite B und Tiefe T der Führungsausnehmung 25 ist an die Größe eines Fadens 10 angepasst, der zur Bildung des geflochtenen Einfadenstents verwendet wird. Die Breite B ist mindestens so groß, wie der Durchmesser des verwendeten Fadens 10, wobei die maximale Breite B durch die Maschenweite W begrenzt ist und somit bevorzugt nicht mehr als 0,9*W beträgt.

Weiterhin ist der Figur 3 zu entnehmen, dass die Breite B der Führungsausnehmung 25 nicht konstant sein muss, sondern die Breite B in der jeweiligen Führungsausnehmung 25 veränderlich ausgebildet sein kann. Bevorzugt ist es, wenn die Breite B ausgehend von der Mitte der Führungsausnehmung 25 zu den Enden der Führungsausnehmung 25, in denen die Führungsausnehmung 25 in die Mantelfläche des Kerns 20 übergeht, zunimmt.

Auf den Flechtkern 2 wird zur Bildung des geflochtenen Einfadenstents der Faden 10 aufgelegt, wobei der Faden 10 ein erstes Ende 11 und ein zweites Ende 12 aufweist. Der Faden 10 kann aus einem metallischen Werkstoff oder einem synthetischen Werkstoff, beispielsweise Polymerfasern, hergestellt sein, und einen Durchmesser zwischen 1 µm und 1000 µm aufweisen, je nachdem für welche Verwendung der tubulär geflochtene Einfadenstent verwendet werden soll.

Besonders bevorzugt ist es, wenn der Faden 10 aus einer Titan-Nickellegierung, beispielsweise Nitinol, hergestellt ist, und einen Durchmesser von ca. 350 µm aufweist.

Der Faden 10 wird, wie nachfolgend anhand der Figuren 2a bis 2g detailliert erläutert wird, ausgehend von dem ersten Ende 11 auf den Flechtkern 2 in spiral- oder helixförmigen Windungen 13 aufgelegt, wobei zur besseren Handhabbarkeit des Fadens 10 an dem zweiten Ende 12 eine Führungseinrichtung 16 angeordnet sein kann. Die Führungseinrichtung 16 kann beispielsweise mittels einer form-, kraft- oder stoffschlüssigen Verbindung mit dem zweiten Ende des Fadens 10 gekoppelt sein, wobei die Führungseinrichtung 16 eine höhere Steifigkeit aufweist als der Faden 10. Insbesondere kann die Führungseinrichtung 16 auch einen größeren Querschnitt, auch mit einem nicht rotationssymmetrischen Querschnitt, bevorzugt einen quadratischen Querschnitt, aufweisen, wodurch die Handhabung des Fadens 10 verbessert ist.

Hierfür wird das erste Ende 11 an dem Flechtkern 2 an dem proximalen Endbereich 21 befestigt und ausgehend von dem ersten Ende 11 mit dem freien Ende 12 auf den Flechtkern 2 aufgelegt, bis ein Umlenkstift 28 der zweiten Umlenkungen 27 in dem distalen Endbereich 22 erreicht ist. Anschließend wird der Faden 10 um einen der Umlenkstifte 28 gelegt und in einer weiteren Windung 13 spiral- oder helixförmig um den Kern 20 zu einer der ersten Umlenkungen 26 in dem proximalen Endbereich 21 geführt. Dieser Vorgang wird wiederholt, bis die Windung 13 beim Legen eine bereits gelegte Windung 13 in einem sogenannten Kreuzungspunkt kreuzt, wobei in diesem Kreuzungspunkt entweder das freie Ende 12 in einer Überführung 14 auf die erste Windung 13 gelegt wird oder in einer Unterführung 15 unter der ersten Windung 13 hindurch geführt bzw. gesteckt wird, wodurch ein Geflecht gebildet wird. Die beiden Windungen 13 schneiden sich in dem Kreuzungspunkt mit einem Kreuzungswinkel α, welcher in Abhängigkeit von einer vorbestimmten Steigung der Windungen 13 ca. 10° ≤ α ≤ 80° und bevorzugt 30° ≤ α ≤ 60° beträgt

Das Geflecht kann in beliebiger Folge gebildet werden, wobei bevorzugt der Faden 10 abwechselnd an einem Kreuzungspunkt in einer Überführung 14 gelegt ist und in eine Unterführung 15 gesteckt wird. Dadurch ergeben sich besonders gute mechanische Eigenschaften für den geflochtenen und tubulären Einfadenstent. Derartige Gewebe können analog als Leinenbindung (plain) bezeichnet werden. Alternativ sind Gewebe mit Köperbindungen (twill), Atlasbindungen (satin) oder kettstarkes Gewebe möglich.

Zum Herstellen des Einfadenstents wird der Faden 10 beabstandet zu dem zweiten Ende 12 von wenigstens einem der Greifer 61, 62 gehalten und anschließend wird der Faden 10 ausgehend von dem zweiten Ende 12 auf die Spule 55 aufgewickelt. Zum Legen des Fadens 10 auf den Flechtkern 2 wird der Flechtkern 2 um die erste Achse 51 gedreht, währenddessen die Spule 55 den Faden 10 unter einer konstanten Fadenspannung in der zweiten Achse 52 entsprechend abwickelt und die Spule 55 entlang der Längsachse X verfahren wird, wodurch die Windung 13 eine entsprechende Steigung erhält.

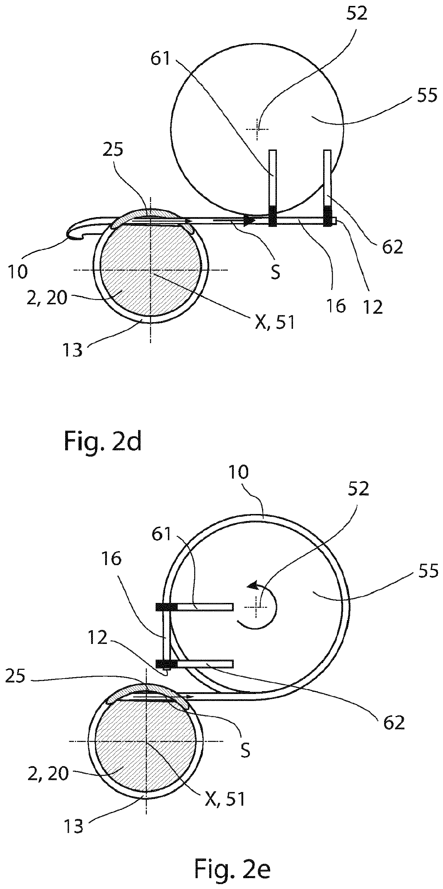

Beim Erreichen eines Kreuzungspunktes, in dem eine Unterführung 15 unter einer bereits abgelegten ersten Windung 13 geschaffen werden soll, wird die bereits gelegte Windung 13 durch ein Haltemittel 65 fixiert. Anschließend wird der Faden 10 vollständig von der Spule 55 abgewickelt und der erste Greifer 61 in einer Steckrichtung S vor der entsprechenden Führungsausnehmung 25 positioniert, wobei der erste Greifer 61 den Faden 10 in der Steckrichtung S koaxial zu der Ausrichtung der Führungsausnehmung positioniert. Der zweite Greifer 62 wird - wie in Figur 2a gezeigt - in Steckrichtung S hinter der Führungsausnehmung 25 zum Entgegennehmen des freien Endes 12 des Fadens 10 positioniert und anschließend wird das freie Ende 12 des Fadens 10 durch die Führungsausnehmung 25 und unter der ersten Windung 13 durchgesteckt und auf der gegenüberliegenden Seite der Führungsausnehmung 25 durch den zweiten Greifer 62 entgegengenommen. Erst wenn der zweite Greifer 62 das freie Ende 12 des Fadens entgegengenommen hat und das freie Ende 12 des Faden 10 sicher hält, lässt der erste Greifer 61, wie in Figur 2c gezeigt ist, den Faden 10 los, damit der Faden 10 durch die Führungsausnehmung und die Unterführung 15 vollständig durchgezogen werden kann. Hierzu kann zunächst der zweite Greifer 62 eine Zugkraft auf das freie Ende 12 des Faden 10 ausüben und anschließend kann, so wie in den Figuren 2d und 2e gezeigt ist, das freie Ende 12 des Fadens 10 auf die Spule 55 aufgespult werden, wodurch der Faden 10 vollständig durch die Führungsausnehmung 25 gezogen wird.

Der erste Greifer 61 und oder zweite Greifer 62, sowie die erste Achse 51 und/oder die zweite Achse 52 können (nicht dargestellte) Kraftsensoren aufweisen, durch die eine an dem Faden 10 anliegende Zugspannung bestimmt werden kann. Besonders bevorzugt ist es, wenn der Kraftsensor ein an der Spule 55 oder an der ersten Achse 51 anliegendes Drehmoment erfasst.

Nach dem vollständigen Durchziehen des Fadens 10 zur Bildung der Unterführung 15 wird die Windung 13 wie bereits zuvor beschrieben weiter auf den Flechtkern 2 gelegt, wobei hinter der Unterführung 15 das Haltemittel 65 positioniert wird und eine Haltekraft auf die Windung 13 aufbringt, damit zum Erzeugen einer weiteren Unterführung 15 beim Abwickeln der Spule 55 die Zugspannung des Fadens 10 in der bereits gelegten Windung 13 nicht verloren geht. Bevorzugt wird beim Legen der Windung 13 der Faden 10 unter einer vorgegebenen und bevorzugt konstanten Zugspannung gehalten.

Der Faden 10 wird so lange in Windungen 13 auf den Flechtkern 2 gelegt, bis das die gelegte Windungen 13 parallel zu einer ersten Windung 13 angeordnet ist. Diese beiden Windungen 13 werden bevorzugt mittels einer Schweißverbindung verbunden und anschließend werden die zwischen der Schweißverbindung und den Enden 11, 12 liegenden freien Endbereiche des Fadens 10 abgeschnitten bzw. entfernt.

Abschließend kann der Flechtkern 2 zusammen mit dem geflochtenen und tubulären Einfadenstent aus der Vorrichtung entnommen werden und durch weitere Verfahrensschritte nachbearbeitet werden. In einem ersten weiteren Verfahrensschritt kann der Einfadenstent bevorzugt auf dem Flechtkern 2 durch Tempern bei ca. 500° thermisch nachbehandelt werden.

Anschließend können die Umlenkstifte 28 von dem Kern 20 gelöst werden und der Einfadenstent kann von dem Kern 20 abgestreift werden. In einem weiteren Verfahrensschritt kann die Oberfläche des Fadens 10 beispielsweise durch Elektropolieren nachbearbeitet werden.

### Bezugszeichenliste

- 2: Flechtkern
- 5: Vorrichtung
- 10: Faden
- 11: erstes Ende
- 12: zweites Ende
- 13: Windung
- 14: Überführung
- 15: Unterführung
- 16: Führungseinrichtung
- 20: Flechtkern
- 21: proximaler Endbereich
- 22: distaler Endbereich
- 25: Führungsausnehmung
- 26: erste Umlenkung
- 27: zweite Umlenkung
- 28: Umlenkstift
- 30: Führungsausnehmung
- 51: erste Achse
- 52: zweite Achse
- 54: Arm
- 55: Spule
- 61: erster Greifer
- 62: zweiter Greifer
- 65: Haltemittel

- B: Breite von 25
- S: Steckrichtung
- T: Tiefe von 25
- X: Längsachse

## Patentansprüche

1. Verfahren zum Herstellen eines Einfadenstents aus einem Faden (10) mit einem ersten Ende (11) und einem zweiten und freien Ende (12), wobei ein Faden (10) ausgehend von dem ersten Ende (11) auf einen Flechtkern (2) mit einer Längsachse (X) spiralförmig in Windungen (13) zwischen wenigstens einer ersten Umlenkung (26) in einem proximalen Endbereich (21) und wenigstens einer zweiten Umlenkung (27) in einem distalen Endbereich (22) gewickelt ist und der Faden (10) abwechselnd an einer Vielzahl von Kreuzungspunkten in eine Überführung (14) über eine Windung gelegt ist und an mindestens einem anderen Kreuzungspunkt in eine Unterführung (15) unter eine gelegte Windung gesteckt wird, beinhaltend die Verfahrensschritte zur Bildung einer Unterführung (15):
- Auflegen einer Windung (13) auf den Flechtkern (20) bis zum Erreichen eines Kreuzungspunktes,
- Einführen und Durchstecken des freien Endes (12) des Fadens (10) in eine Steckrichtung (S) im Wesentlichen senkrecht zu der Längsachse (X) in eine Führungsausnehmung (25) des Flechtkerns (2) und
- Entgegennehmen und Durchziehen des Fadens (10) durch die Führungsausnehmung (25),
**dadurch gekennzeichnet, dass** das freie Ende (12) des Fadens (10) in der Steckrichtung (S) entlang einer Geraden, welche sekantial zu dem Flechtkern (2) angeordnet ist, durch die Führungsausnehmung (25) gesteckt wird.

2. Verfahren nach Anspruch 1,
**dadurch gekennzeichnet, dass** der Faden (10) durch die Führungsausnehmung (25) so lange durchgezogen wird, bis die Windung (13) des Fadens (10) mit einer vorgegebenen Spannung auf dem Flechtkern (2) gelegt ist.

3. Verfahren nach Anspruch 1 oder 2,
**dadurch gekennzeichnet, dass**
die Steckrichtung (S) parallel zur der Führungsausnehmung (25) ausgerichtet ist.

4. Verfahren nach einem der vorgenannten Ansprüche,
**dadurch gekennzeichnet, dass**
ein erster Greifer (61) den Faden (10) in einem Abstand zu dem freien Ende (12) hält und das freie Ende (12) in die Führungsausnehmung (25) in der Steckrichtung (S) einsteckt und/oder dass ein zweiter Greifer (62) das freie Ende (12) des Fadens (10) in Steckrichtung (S) hinter der Führungsausnehmung (25) entgegennimmt.

5. Verfahren nach Anspruch 4,
**dadurch gekennzeichnet, dass**
der erste Greifer (61) den Faden (10) mindestens so lange hält, bis der zweite Greifer (62) das freie Ende (12) des Fadens (10) hält.

6. Verfahren nach einem der vorgenannten Ansprüche,
**dadurch gekennzeichnet, dass**
der Faden (10) nach dem Durchziehen durch die Führungsausnehmung (25) auf dem Flechtkern (2) gehalten ist.

7. Verfahren nach einem der vorgenannten Ansprüche,
**dadurch gekennzeichnet, dass**
das Durchziehen des Fadens (10) ausgehend von dem zweiten freien Ende (12) durch ein Aufwickeln des Fadens (10) auf einer Spule (55) um eine zweite Achse (52) erfolgt.

8. Verfahren nach Anspruch 7,
**dadurch gekennzeichnet, dass**
ein vollständiges Abwickeln des Fadens (10) von der Spule (55) vor dem Ein- und Durchstecken des freien Endes (12) des Fadens (10) zur Bildung der Unterführung (15) erfolgt.

9. Flechtkern (2), aufweisend:
- einen Kern (20) mit einer Längsachse (X) und einem proximalen Endbereich (21) und einem distalen Endbereich (22), und
- wenigstens eine erste Umlenkung (26) und wenigstens eine zweite Umlenkung (27),
- wobei in dem proximalen Endbereich (21) die wenigstens eine erste Umlenkung (26) ist und in dem distalen Endbereich (22) die wenigstens eine zweite Umlenkung (27), und
- wobei zwischen dem proximalen Endbereich (21) und dem distalen Endbereich (22) eine Vielzahl von Führungsausnehmungen (25) angeordnet sind, **dadurch gekennzeichnet, dass**
- die jeweilige Führungsausnehmung (25) als sekantial verlaufende Nut oder Ausnehmung im Wesentlichen senkrecht zur der Längsachse (X) in die Mantelfläche des Kerns (20) eingearbeitet oder eingeformt ist.

10. Flechtkern (2) nach Anspruch 9,
**dadurch gekennzeichnet, dass**
über den Umfang des Kerns (20) in einer Ebene senkrecht zu der Längsachse (X) eine Anzahl (j) von Führungsausnehmungen (25) umfangssymmetrisch angeordnet sind, und/oder dass parallel und beabstandet zu der Längsachse eine Anzahl (k) von Führungsausnehmungen (25) angeordnet ist.

11. Vorrichtung (5) zum Durchführen des Verfahrens nach zumindest Anspruch 4 und einem der Ansprüche 2, 3 oder 5 bis 8 mit einem Flechtkern (2) mit den Merkmalen nach einem der Ansprüche 9 bis 10, aufweisend
- eine erste Achse (51) und eine zweite Achse (52),
- wenigstens einen Arm (54) mit einem ersten Greifer (61), der eingerichtet ist, einen Faden (10) zu greifen und zu halten, und
- wenigstens eine Spule (55)
- wobei der Flechtkern (2) in der ersten Achse (51) angeordnet ist, **dadurch gekennzeichnet, dass**
- der Arm (54) und die wenigstens eine Spule (55) um die zweite Achse (52) drehbar angeordnet sind,
- wobei der erste Greifer (61) den Faden (10) in einem Abstand zu dem freien Ende (12) halten kann und das freie Ende (12) in die Führungsausnehmung (25) in der Steckrichtung (S) einstecken kann.

12. Vorrichtung (5) nach Anspruch 11,
**dadurch gekennzeichnet, dass**
der wenigstens eine Arm (54) den ersten Greifer (61) und einen zweiten Greifer (62) umfasst, die parallel und beabstandet zueinander angeordnet sind und zusammen mit der Spule (55) um die zweite Achse (52) drehbar sind.

13. Vorrichtung (5) nach einem der Ansprüche 11 oder 12, **dadurch gekennzeichnet, dass** der Arm (54) relativ zu der ersten Achse (51) beweglich angeordnet ist und/oder dass
die erste Achse (51) und die zweite Achse (52) parallel zueinander ausgerichtet sind, oder dass die erste Achse (51) und die zweite Achse (52) senkrecht zueinander stehen.

## Claims

1. Method for manufacturing a single-filament stent from a filament (10) having a first end (11) and having a second and free end (12), wherein a filament (10), starting from the first end (11), is wound spirally into windings (13) onto a braiding core (2), which has a longitudinal axis (X) between at least one first deflection (26) in a proximal end region (21) and at least one second deflection (27) in a distal end region (22), and the filament (10) is placed, alternately on a plurality of crossover points, into an upper viaduct (14) over a winding and, at at least one further crossover point, is inserted into a lower viaduct (15) under a winding, including the method steps for forming a lower viaduct (15):
- applying a winding (13) onto the braiding core (20) until a crossover point is reached,
- inserting into and piercing with the free end (12) of the filament (10), in an insertion direction (S) which extends essentially vertically to the longitudinal axis (X), a guide cutout (25) of the braiding core (2), and
- receiving and pulling the filament (10) through the guide cutout (25)
**characterized in that** the free end (12) of the filament (10) is inserted, in an insertion direction (S) which extends along a line arranged secantially in relation to the braiding core (2), through the guide cutout (25).

2. Method in accordance with claim 1,
**characterized in that**
the filament (10) is pulled through the guide cutout (25) until the winding (13) of the filament (10) has been applied onto the braiding core (2) with a specified tension.

3. Method in accordance with claim 1 or 2,
**characterized in that**
the insertion direction (S) extends parallel to the guide cutout (25).

4. Method in accordance with any of the preceding claims,
**characterized in that**
a first gripper (61) holds the filament (10) at a distance to the free end (12) and inserts the free end (12), in the insertion direction (S), into the guide cutout (25) and/or **in that** a second gripper (62) receives, in the insertion direction (S), the free end (12) of the filament (10) behind the guide cutout (25).

5. Method in accordance with claim 4,
**characterized in that**
the first gripper (61) holds the filament (10) at least until the second gripper (62) has taken hold of the free end (12) of the filament (10).

6. Method in accordance with any of the preceding claims
**characterized in that**
the filament (10) is held on the braiding core (2) after having been pulled through the guide cutout (25).

7. Method in accordance with any of the preceding claims
**characterized in that** pulling through the filament (10), starting from the second free end (12), is carried out by winding the filament (10) onto a spool (55) around a second axis (52).

8. Method in accordance with claim 7,
**characterized in that**
a complete unwinding of the filament (10) from the spool (55) is carried out before the insertion of and piercing with the free end (12) of the filament (10), in order to form the lower viaduct (15).

9. Braiding core (2), comprising:
- a core (20) with a longitudinal axis (X) and having a proximal end region (21) and a distal end region (22), and
- at least one first deflection (26) and at least one second deflection (27),
- wherein the at least one first deflection (26) is in the proximal end region (21) and the at least one second deflection (27) is in the distal end region (22), and
- wherein a plurality of guide cutouts (25) are arranged between the proximal end region (21) and the distal end region (22),
**characterized in that**
- each of the guide cutouts (25) is machined or formed into the outer surface of the core (20) as a secantially extending groove or cutout arranged essentially vertically to the longitudinal axis (X).

10. Braiding core (2) in accordance with claim 9,
**characterized in that** a plurality (j) of guide cutouts (25) are arranged, circumferentially and symmetrically in a plane extending vertically to the longitudinal axis (X), around the circumference of the core (20), and/or **in that** a plurality (k) of guide cutouts (25) are arranged parallel and at a distance to the longitudinal axis (X).

11. Apparatus (5) for carrying out the method in accordance with at least claim 4 and one of claims 2, 3 or 5 to 8, with a braiding core (2) having the features in accordance with either of claims 9 or 10, comprising:
- a first axis (51) and a second axis (52),
- at least one arm (54) with a first gripper (61), which is implemented to grip and hold a filament (10), and
- at least one spool (55)
- wherein the braiding core (2) is arranged in the first axis (51),
**characterized in that**
- the arm (54) and the at least one spool (55) are rotatably arranged around the second axis (52),
- wherein the first gripper (61) can hold the filament (10) at a distance to the free end (12) and can insert the free end (12), in the insertion direction (S), into the guide cutout (25).

12. Apparatus (5) in accordance with claim 11,
**characterized in that**
the at least one arm (54) includes the first gripper (61) and a second gripper (62), which are arranged parallel and at a distance to each other and which are rotatable, together with the spool (55), around the second axis (52).

13. Apparatus (5) in accordance with either of claims 11 or 12, **characterized in that**
the arm (54) is arranged movably in relation to the first axis (51) and/or in that the first axis (51) and the second axis (52) extend parallel to each other or in that the first axis (51) and the second axis (52) extend vertically to each other.

## Revendications

1. Procédé pour réaliser un stent à fil unique avec un fil (10) ayant une première extrémité (11) et une seconde extrémité libre (12),
selon lequel
- on enroule le fil (10) partant de la première extrémité (11) sur un mandrin de tressage (2) d'axe longitudinal (X), suivant une forme en spirale selon les spires (13) entre au moins une première déviation (26) dans une zone d'extrémité proximale (21) et au moins une seconde déviation (27) dans une zone d'extrémité distale (22), et
- dans un passage (14) sur une spire, on place le fil (10) alternativement en plusieurs points de croisement, et
- en au moins un autre point de croisement, on le met dans un passage (15) sous une spire posée,
procédé comprenant les étapes de formation d'un passage en dessous (15) :
- pose d'une spire (13) sur le mandrin de tressage (2) jusqu'à atteindre un point de croisement,
- introduire et faire passer l'extrémité libre (12) du fil (10) dans une direction d'enfilage (S) qui est pratiquement perpendiculaire à l'axe longitudinal (X) dans un évidement de guidage (25) du mandrin de tressage (2), et
- reprendre et faire passer le fil (10) à travers l'évidement de guidage (25),
procédé **caractérisé en ce que**
l'extrémité libre (12) du fil (10) est disposée dans la direction d'enfilage (S) selon une droite sécante au mandrin de tressage (2) en enfilant à travers l'évidement de guidage (25).

2. Procédé selon la revendication 1,
**caractérisé en ce que**
on tire le fil (10) à travers l'évidement de guidage (25) jusqu'à ce que la spire (13) du fil (10) soit mise à une tension prédéfinie sur le mandrin de tressage (2).

3. Procédé selon la revendication 1 ou 2,
**caractérisé en ce que**
la direction d'extension (S) est parallèle à l'évidement de guidage (25).

4. Procédé selon l'une des revendications précédentes,
**caractérisé en ce que**
une première pince (61) tient le fil (10) à une distance de l'extrémité libre (12) et engage la première extrémité (12) dans l'évidement de guidage (25) dans la direction d'enfilage (S) et/ou une seconde pince (62) reprend l'extrémité libre (12) du fil (10) dans la direction d'enfilage (S) derrière l'évidement de guidage (25).

5. Procédé selon la revendication 4,
**caractérisé en ce que**
la première pince (61) tient le fil (10) au moins jusqu'à ce que la seconde pince (62) tienne l'extrémité libre (12) du fil (10).

6. Procédé selon l'une des revendications précédentes,
**caractérisé en ce que**
après avoir été tiré à travers l'évidement de guidage (25), le fil (10) est tenu sur le mandrin de tressage (2).

7. Procédé selon l'une des revendications précédentes,
**caractérisé en ce que**
on tire le fil (10) à partir de la seconde extrémité (12) en enroulant le fil (10) sur une bobine (55) autour d'un second axe (52).

8. Procédé selon la revendication 7,
**caractérisé en ce que**
on déroule complètement le fil (10) de la bobine (55) avant d'engager et de faire traverser l'extrémité libre (12) du fil (10) pour former le passage en dessous (15).

9. Mandrin de tressage (2) comprenant :
- un mandrin (20) d'axe longitudinal (X) avec une zone d'extrémité proximale (21) et une zone d'extrémité distale (22), et
- au moins la première déviation (26) et au moins une seconde déviation (27),
- au moins la première déviation (26) est dans la zone d'extrémité (21) et au moins la seconde déviation (27) est dans la zone d'extrémité distale (22), et
- un nombre d'évidements de guidage (25) entre la zone d'extrémité proximale (21) et la zone d'extrémité distale (22),
noyau **caractérisé en ce que**
chaque évidement de guidage (25) est usiné ou formé comme rainure sécante ou évidement pratiquement perpendiculaire à l'axe longitudinal (X) dans la surface-enveloppe du mandrin (20).

10. Noyau de tressage (2) selon la revendication 9,
**caractérisé en ce que**
un nombre (j) d'évidements (25) est prévu de manière symétrique en rotation sur la périphérie du mandrin (20) dans un plan perpendiculaire à l'axe longitudinal (X),
et/ou un nombre (k) d'évidements de guidage (25) est prévu parallèlement et de façon écartée par rapport à l'axe longitudinal.

11. Dispositif (5) pour la mise en oeuvre du procédé selon au moins la revendication 4 et l'une des revendications 2, 3 ou 5 à 8 avec un noyau de tressage (2) ayant les caractéristiques de l'une des revendications 9 à 10, comprenant :
- un premier axe (51) et un second axe (52),
- au moins un bras (54) avec une première pince (61) pour prendre et tenir un fil (10), et
- au moins une bobine (55),
- le noyau de tressage (2) étant installé selon le premier axe (51), dispositif **caractérisé en ce que**
- le bras (54) et au moins la bobine (55) sont installés à rotation autour du second axe (52),
- la première pince (61) pouvant tenir le fil (10) à une distance de la première extrémité (12) et l'extrémité libre (12) pouvant s'enfiler dans l'évidement de guidage (25) selon la direction d'enfilage (S).

12. Dispositif (5) selon la revendication 11,
**caractérisé en ce que**
au moins un bras (54) comprend la première pince (61) et une seconde pince (62), ces pinces étant parallèles et écartées l'une de l'autre et montées à rotation avec la bobine (55) autour du second axe (52).

13. Dispositif (5) selon l'une des revendications 11 ou 12,
**caractérisé en ce que**
- le bras (54) est monté mobile par rapport au premier axe (51), et/ou
- le premier axe (51) et le second axe (52) sont orientés parallèlement l'un à l'autre, ou
- le premier axe (51) et le second axe (52) sont perpendiculaires l'un à l'autre.
